# EUROPEAN PATENT APPLICATION

(11) **EP 4 748 347 A1**
(43) Date of publication of application: **27.05.2026**
(21) Application number: 24290043.9
(22) Date of filing: 22.11.2024
(51) Int. Cl.: A61F 2/16, B29D 11/02, G02C 1/00

(54) **INTRAOCULAR LENS WITH OPTICAL CENTRE OFFSET WITH RESPECT TO ITS GEOMETRICAL CENTRE**

(71) Applicant: D Gatinel, 75019 Paris (FR)
(72) Inventor: Gatinel, Damien, 75006 Paris (FR)
(74) Representative: Wohlmuth, Johannes

(57) **Abstract**

An intraocular lens (21) comprising haptics (30) configured to position the intraocular lens (21) in a defined orientation in the eye, wherein the haptics (30) define a geometrical centre point (C) of the intraocular lens (21), wherein the intraocular lens (21) comprises further an optical lens (25) with an optical centre point through which the optical axis of the optical lens (25) extends, wherein the optical lens (21) is arranged such with respect to the haptics (30) that the optical centre point is offset horizontally and vertically with respect to the geometrical centre point (C).

## Description

### Technical Field

The present invention relates to the field of intraocular lenses.

### Prior art

The purpose of inserting implants into the human eye is to correct ametropia (phakic implants) or to replace the lens that is clouded or likely to be removed as part of a refractive surgery known as the clear lens (pseudophake implant). The quality of intraocular lenses (IOL) as phakic or pseudophake implants have signifcantly improved over the last decades so that the result of lens-based surgeries nowadays are very good. Nevertheless, there remain still some issues.

Some patients report an annoying adverse reaction after cataract surgery, perceived as a dark and/or bright crescent in the temporal visual field. This phenomenon, called dysphotopsia, is attributed to a side effect of the nasal part of the implant, which receives oblique rays and refracts them irregularly at the level of the peripheral nasal retina.

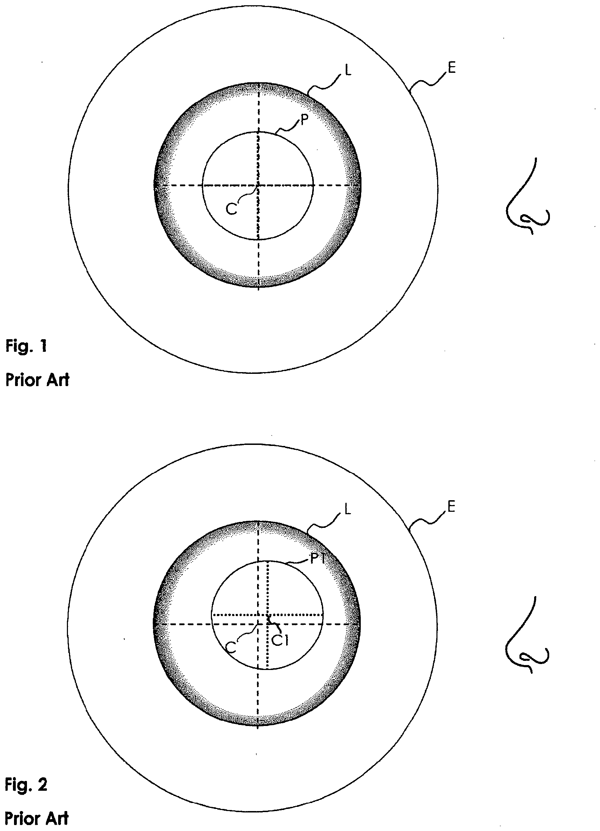

Another unrelated problem is that the centre of the pupil is actually not aligned with the centre of the limbus. This can cause the optics to shift and reduce the quality of vision when the optics are at high power. To illustrate this, Fig. 1 shows the eye or eyeball E, the limbus L with the iris I and the pupil P. Fig. 1 shows when the centre point C of the limbus L is aligned with the centre point of the pupil P. When the centre point C of the limbus L is offset with respect to the centre point C1 of the pupil P1, i.e. with respect to the optical centre of the eye is shown in Fig. 2.

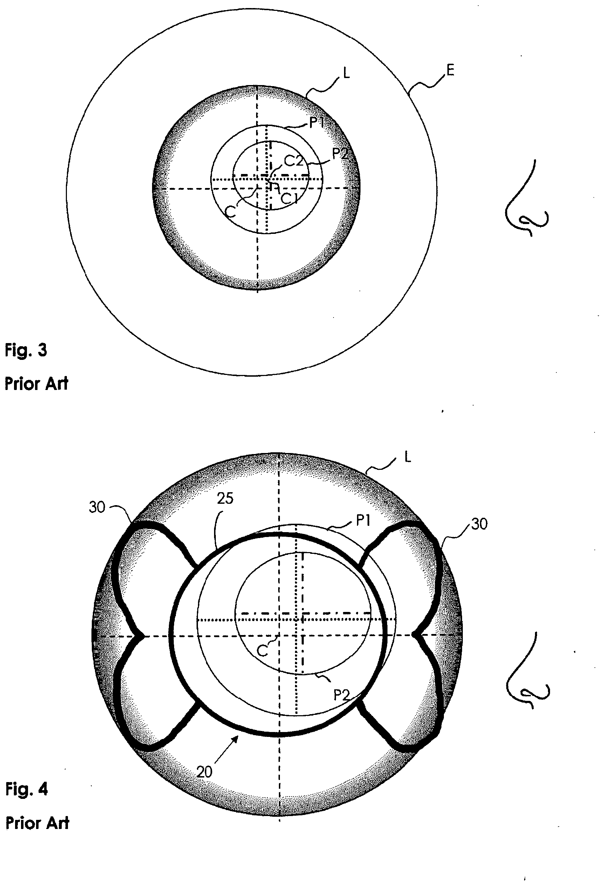

In the article "The Form of the Human Pupil" from Harry J. Wyatt, published in Vision Res. Vol 35, No 14, pp. 2021-2036 in 1995, this offset has been studied. It showed that many people have this offset and that there is a statistical tendency that the offset of the optical centre C1 of the pupil P is more often nasally and upwards. It was further shown that this offset further increases for bright light conditions compared to dark light conditions. While Fig. 2 shows the pupil P1 and its centre C1 under dark light conditions. Fig. 3 shows this centre point C2 of the pupil P2 under bright light conditions. Simiar findings have been confirmed in the article "One-eye centration of soft contact lenses" by Grace Walther et al published in Ophthalmic and physiological optics on 12 January 2024.

This offset between the geometrical centre of the limbus L and the centre of the pupil P is normally neglected for the shape of the phakic and pseudophakic lOLs. All standard (non-customized) IOLs and even most of the customized lOLs have their optical centre centred with respect to the geometrical centre of the IOL defined by the haptics. Therefore, most of the IOLs inserted in the eye of the patient have their optical centre aligned with the geometrical centre of the eye E or the limbus L, but not aligned with the optical centre C1. Even if this misalignment is minor in magnitude, it can significantly reduce optical quality and increase the likelihood of dysphotopsia phenomena.

Therefore, US4441217, US2008/0269881, EP2575680 and WO2022/219214A1 propose actually to align the optical centre of the IOL to the to the centre of the pupil P. In other words, the optical centre of the IOL (defined by the lens) is offset with respect to the geometrical centre of the IOL (defined by the haptics). This requires however a customization of the IOL for each eye of the patient which is very time and resource consuming.

### Brief summary of the Invention

it is the object of the invention to provide improved lOLs for cataract surgery.

According to the invention, this object is solved by an IOL according to one of the independent claims.

According to the invention, this object is solved by a non-customized intraocular lens comprising haptics configured to position the intraocular lens in a pre-defined orientation in the eye, wherein the haptics define a geometrical centre point of the intraocular lens, wherein the intraocular lens comprises further an optical lens with an optical centre point through which the optical axis of the optical lens extends, wherein the optical lens is arranged such with respect to the haptics that the optical centre point is offset horizontally and vertically with respect to the geometrical centre point.

According to the invention, this object is solved by a customizable intraocular lens blank comprising haptics configured to position the intraocular lens in a pre-defined orientation in the eye, wherein the haptics define a geometrical centre point of the intracolar lens, wherein the intraocular lens comprises further an optical lens blank with an optical centre point through which the optical axis of the optical lens blank extends, wherein the optical lens blank is configured to be customizable to a patient wherein the optical lens blank is arranged such with respect to the haptics that the optical centre point is offset horizontally and vertically with respect to the geometrical centre point.

According to the invention, this object is solved by a customized intraocular lens comprising haptics configured to position the intraocular lens in a pre-defined orientation in the eye, wherein the haptics define a geometrical centre point of the intracolar lens, wherein the intraocular lens comprises further an optical lens with an optical centre point through which the optical axis of the optical lens extends, wherein the optical lens is customized to a patient, wherein the optical lens is arranged such with respect to the haptics that the optical centre point is offset horizontally and vertically with respect to the geometrical centre point, wherein the offset arrangement of the optical centre point of the optical lens with respect to the geometrical centre of the haptics is non-costumized.

According to the invention, this object is solved by Pair of intraocular lenses comprising a left intraocular lens and a right intraocular lens, wherein the left intraocular lens comprises left haptics configured to position the left intraocular lens in a defined orientation in a left eye of a patient, wherein the left haptics define a left geometrical centre point of the left intracolar lens, wherein the left intraocular lens comprises further a left optical lens with a left optical centre point through which the optical axis of the left optical lens extends, wherein the left optical lens is arranged such with respect to the left haptics that the left optical centre point is offset horizontally and vertically with respect to the left geometrical centre point, wherein the right intraocular lens comprises right haptics configured to position the right intraocular lens in a defined orientation in a right eye of the patient, wherein the right haptics define a right geometrical centre point of the right intracolar lens, wherein the right intraocular lens comprises further a right optical lens with a right optical centre point through which the optical axis of the right optical lens extends, wherein the right optical lens is arranged such with respect to the right haptics that the right optical centre point is offset horizontally and vertically with respect to the right geometrical centre point, wherein the right intraocular lens and the left intraocular lens are intraocular lenses with a non-customized offset between the optical centre point and the geometrical centre point. Preferably the IOLs are non-customized intraocular lenses or customizable intraocular lens blanks or customized intraocular lenses with non-customized offset.

According to the invention, this object is solved by multiple pairs comprising two, three or more pairs as described above, wherein the two, three or more pairs are identical pairs.

According to the invention, this object is solved by multiple non-customized intraocular lenses as described above, wherein the multiple non-customized intraocular lenses are identical.

While in the state of the art the offset between the geometrical centre of the haptics and the optical centre of the optical lens have been customized to the measured offset of a patient between the centre of the limbus and the centre point of the pupil in order to optimize the optical system of the eye after the surgery. However, it was found out that a standard offset in a horizontal and a vertical direction applied for all patients can improve as well the optical alignment (in a statistical sense), and more importantly improve the problem of dysphotopsia for all patients (also the ones with other offsets).

It has been further found out that the use of distinct lOLs for the left and right eye with non-customized offsets improved significantly the optics and the problem of dysphotopsia, even though standardized IOLs or customized IOLs with standard offset were used.

The object is further solved by a method for selecting an intraocular lens as described above for a patient or for selecting a pair of intraocular lenses as described above for a patient, wherein the intraocular lens or the pair is selected independent from the pupil centre of the patient.

An IOL customized on the pupil centre offset of a patient is very complex to manufacture and the obtained improvement is often only minimal so that in practise such an optical offset customization has not been done. However, as the inventor found out that a standard vertical and horizontal offset has a beneficial effect for all patients, IOLs with non-customized offsets for all patients make the implementation very easy with a further improvement of the optical quality of the IOL for all patients.

The dependant claims refer to further advantageous embodiments.

In one embodiment, the optical centre point is offset nasaly with respect to the geometrical centre point. Preferably, the optical centre point is offset nasaly with respect to the geometrical centre point by an nasal offset larger than 0.1mm, preferably larger than 0,15 mm and/or smaller than 0,35mm, preferably smaller than 0,30 mm.

In one embodiment, the optical centre point is upwards with respect to the geometrical centre point. Preferably, the optical centre point is offset upwards with respect to the geometrical centre point by a vertical offset larger than 0,05mm, preferably larger than 0,08 mm and/or smaller than 0,25mm, preferably smaller than 0,20 mm.

In one embodiment, the optical centre point is offset nasaly and upwards with respect to the geometrical centre point.

The offset nasally and/or upwards fits statistically more patients than the optical center aligned with the geometrical centre point. Therefore, statistically, the patients should have an improved optical alignment of the optical lens of the IOL with the pupil centre leading to an improved total optical system after surgery. However, the nasal and/or upwards offset have in addition a beneficial effect to avoid dysphotopsia even for patients which do not necessarily have a nasal or upwards offset between the pupil centre point and the centre point of the limbus. This might be explained by the fact that the more nasal and/or more upwards position move the IOL further away from oblique light rays from the temporal side of the face. These oblique temporal rays reach thus less the nasal edge of the IOL due to its nasal and upwards movement so that the temporal edge effect is seen less. Thus, the nasal and/or upwards offset leads not only for patients with nasal and upwards offset of the pupil centre to an improvement of their optical system after surgery, but leads in general to a reduction of dysphotopsia for all patients, even those who do not show a nasal or upwards offset of their pupil centre.

In one embodiment, the nasal offset is larger than the upwards offset.

In one embodiment, the left intraocular lens is different from the right intraocular lens.

In one embodiment, the offset of the left intraocular lens is different from the offset of the right intraocular lens.

In one embodiment, the left intraocular lens is different from the right intraocular lens such that the right intralcular lens cannot be obtained by a rotation by 180° of the left intraocular lens around the left geometrical centre point.

In one embodiment, the left optical centre point is offset nasaly and upwards with respect to the left geometrical centre point, wherein the right optical centre point is offset nasaly and upwards with respect to the right geometrical centre point.

In one embodiment, the nasal offset of the left optical centre point is different from the nasal offset of the right optical centre point and/or the vertical offset of the left optical centre point is different from the vertical offset of the rigt optical centre point.

In one embodiment, the intraocular lens or the pair with the horizontal and vertical offset is selected without measuring the pupil centre offset of the patient (including analogue measurements) or without considering such a measurement for the selection of the horizontal and vertical offset of the intraocular lens or pair.

Other embodiments according to the present invention are mentioned in the appended claims and the subsequent description of an embodiment of the invention.

### Brief description of the Drawings

Figure 1 shows schematically an eye with a cocentrically alligned limbus and pupil as know in the prior art.
Figure 2 shows schematically an eye with a statistical offset of the pupil centre from the limbus centre under dark light conditions as know in the prior art.
Figure 3 shows schematically an eye with a statistical offset of the pupil centre from the limbus centre under dark and bright light conditions as know in the prior art.
Figure 4 shows schematically an eye with a statistical offset of the pupil centre from the limbus centre under dark and bright light conditions as know in the prior art with an IOL of the state of the art.
Figure 5 shows an IOL according to the invention with an offset of the optical axis with respect to the geometrical axis of the IOL.
Figure 6 shows schematically a pair of eyes with a statistical offset of the pupil centre from the limbus centre under dark and bright light conditions as know in the prior art.
Figure 7 shows a pair of IOLs according to the invention, both with an offset of the optical axis with respect to the geometrical axis of the IOL.
Figure 8 shows a light simulation for temporal oblique light rays for an eye with a natural lens.
Figure 9 shows a light simulation for temporal oblique light rays for an eye with an implanted IOL according to the state of the art.
Figure 10 shows a light simulation for temporal oblique light rays for an eye with an implanted IOL according to an aspect of the invention.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

Before starting describing the IOL according to the invention, some terms shall be defined/described.

First, the anatomy of the eye is quickly resumed. Light is focused into the eye through the cornea, a clear, dome-shaped front portion of the eye called the cornea. Behind the cornea is a fluid-filled space called the anterior chamber. The fluid is called aqueous humor. Behind the anterior chamber is the eye's iris (the colored part of the eye) and the dark hole in the middle called the pupil. Muscles in the iris dilate (widen) or constrict (narrow) the pupil to control the amount of light reaching the back of the eye. The limbus L corresopnds to the transition between the cornea and the sclera. Directly behind the pupil sits the lens. The lens focuses light toward the back of the eye. The lens changes shape to help the eye focus on objects up close. Small fibers called zonules are attached to the capsule holding the lens, suspending it from the eye wall. The lens is surrounded by the lens capsule, which is left in place when the lens is removed during IOL surgery. Normally, the pseudophakic intraocular lenses go inside the capsule, where the natural lens was. The phakic lOLs are inserted into the eye sulcus.

The centre point C of the limbus L is the centre point of the shape of the limbus L or of the outer circumference of the iris. If this shape is circular, it is simply the centre point of the circle. If this shape is elipsoidal, it would correspond to the centre point of the ellipse. If this shape is different, it would correspond to the centre of gravity of the shape.

Each human has a pair of eyes comprising a right eye and a left eye. A human body can be devided along the median plane (or mid-saggital plane) in a right body half and a left body half. The left and right is meant from the perspective of the human himself/herself. The left body half contains thus the heart. The left eye refers to the eye arranged in the left body half. The right eye refers thus to the eye arranged in the right body half.

A horizontal direction is a direction perpendicular to the median plane. The horizontal direction can be divided in a right and left direction. With respect to each body half, the horizontal direction can also be divided in the two opposed directions: nasal direction and temporal directoin. The nasal direction is a direction from an eye of the human towards the nose, while the temporal direction is a direction opposite to the nasal direction and/or away from the nose. The nasal side of the eyes E shown in the Figures is represented by a small symbol of a nose.

A vertical direction is a direction parallel to the median plane and the frontal plane (or coronal plane) of the human. The vertical direction can be divided in the two opposed directions : top direction and bottom direction. The top direction is from the feet towards the head. The bottom direction is from the head towards the feet. The top direction can also be called upper direction or referred to by adjectives like above, upwards, superior, etc.. The upper side of the eyes E shown in the Figures corresponds to the upper side of the drawing sheet. The bottom direction can also referred to by adjectives like below, downwards, inferior, etc.. The lower side of the eyes E shown in the Figures corresponds to the lower side of the drawing sheet.

A sagittal horizontal direction is a direction perpendicular to the horizontal direction or is a direction parallel to the horizontal plane and to the median plane. The saggital horizontal direction can be divided in the two opposed directions: back direction and front direction. The front direction can also be referred to by the adjectives anterior, front, etc.. From the perspective of the eye of the human, the front direction might also be called the outside direction thus the direction pointing out of the body or out of the head/eye The back direction can also be referred to by the adjectives posterior, back, etc.. From the perspective of the eye of the human or of the position of an inserted IOL, the back direction might also be called the interior direction thus the direction pointing in the body or in the head.

An intraocular lens (IOL) is an artificial lens that is implanted in the eye during IOL surgery. The IOL could be a phakic IOL to correct for an ametropia. The IOL could also be a pseudophakic IOL to replace the natural lens during a cataract surgery.

IOL surgery refers in general to the insertion or implantation of an IOL in the eye. IOL surgery can be a cataract surgery, a refractive lens exchange or a ametropia correction surgery.

During cataract surgery, an IOL replaces the natural lens of the eye by an IOL. It is designed to restore clear vision by focusing light onto the retina. In cataract surgery, after the crystalline lens is removed, the implant is placed in the capsular bag, where it is secured by haptics, the design of which can vary depending on the implant model.

In the case of ametropia correction without removing the crystalline lens, a phakic implant is inserted in the eye. Most phakic implants are placed in the ciliary sulcus. Their power is determined by a prior refractive calculation and their size is chosen according to an abacus that generally takes into account certain dimensions of the posterior or anterior segments of the eye. Phakic lOLs have generally rectangular haptics and are inserted into the ocular sulcus, posterior to the iris. Phakic implants for the sulcus come in various sizes, as it is critical for the optic of the implant to be positioned within a safe vault zone, maintaining adequate distance from both the crystalline lens and the posterior surface of the cornea. Other designs for phakic IOLs involve angle-supported lenses, for which the haptics are inserted in the angle formed by the anterior iris surface and posterior corneal surface. Angle-supported anterior chamber phakic lOLs must precisely match the dimensions of the anterior chamber. An undersized IOL can lead to complications such as rotation or decentration, while an oversized IOL may cause pupil distortion and iris atrophy. Similarly, sulcus-fixated posterior chamber phakic lOLs require accurate sizing to avoid contact with the natural lens, which can lead to cataract formation if the IOL is too small, or to angle closure and iris displacement if the IOL is too large. The optic of these IOLs should be centered on the line of sight to avoid the induction of higher order aberrations such as coma.

A customized IOL is an IOL which is customized to measurements of the eye of a patient. Thus, the eye of the patient is here measured before the IOL surgery and then the customized IOL is manufactured based on the measurements. The customized IOLs are normally manufactured by additive manufacturing, by removing manufacturing. Removing manufacturing is the removal of material from a IOL blank, e.g. by machining or laser processing.

A non-customized IOL is an IOL which can be used for a group of patients, i.e. more than one, two, three or more patients, preferably, more than 10, 20, 50, 100, 500, 1000 or 10000 patients. The non-customized IOL is also called standard IOL or pre-designed IOL, i.e. pre-designed according to standard patient groups and not designed according to specific patient measurements. The non-customized IOL is preferably manufactured by molding. However, non-customized IOLs can also be manufactured differently than by molding. Non-customized IOLs exist normally for different lens powers so that a surgeon can select the lens power required for a specific patient.

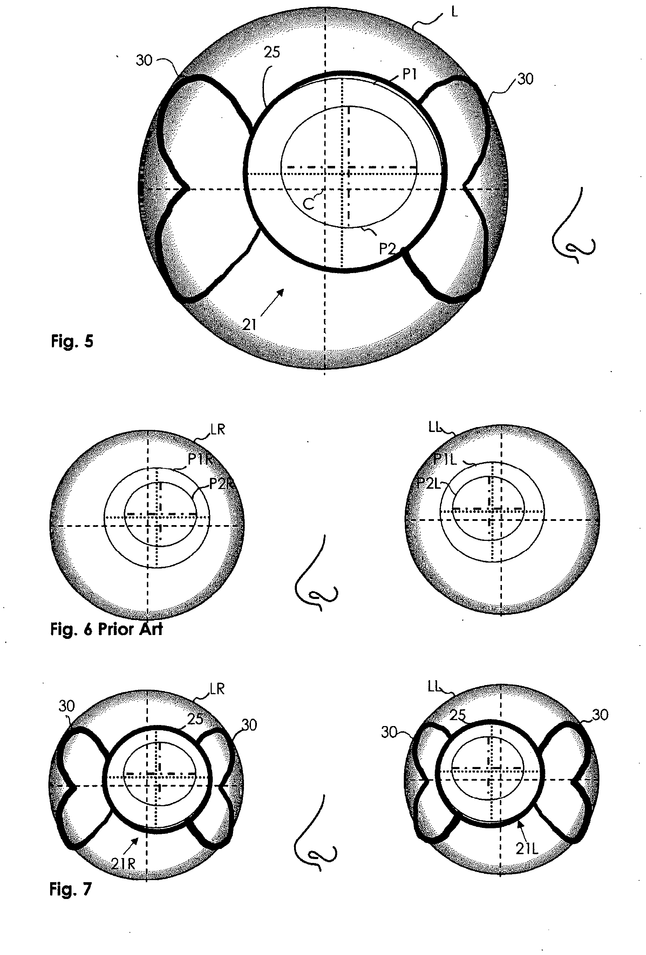

Fig. 5 shows an embodiment of an IOL according to the invention. The IOL 21 comprises haptics 30 and an optical lens 25.

The optical lens 25 has shape which, when inserted in the eye of the patient, causes light coming from the outside of the eye (and having passed the cornea) to change the focal behaviour of the light. Each optical lens 25 has preferably an optical center point or an optical axis. The optical center point is the point in which the optical axis of the optical lens 25 cuts a projection plane of the optical lens 25 or the IOL 21. For rotationally symmetric IOLs like spherical IOLs, aspherical IOLs, most multifocal eccentric IOLs, the symmetry point/axis of the rotational symmetry corresponds to the optical center point or optical axis of the optical lens 25. However, also for non-symmetrical lOLs like for toric IOLs, spirally formed IOLs, there is a well-defined optical center point of the optical lens 25. For example, for an spirally formed IOL, the mathematical starting point of the spiral could be considered as the centerpoint. For a toric IOL, the optical center point can be defined as the intersection between the steepest meridian and the flattest meridian. For asymmetrical optics (such as multifocal implants with a decentered inferior addition), the optical center is generally considered to be the geometric center of the optic, particularly for the standard, non-customized implants currently in production . The optical lens 25 can be any type of IOL lens like phakic, pseudophakic, monofocal, bifocal, trifocal, multifocal, refractive, diffractive, extended depth of focus lOLs, spheric, aspheric, toric, etc.. The optical lens 25 has perferably a rotationally symmetric shape.

The haptics 30 are the supporting structures that hold the IOL 21 and/or the optical lens 25 in place within the eye E.

The haptics 30 have a geometrical centre. The geometrical centre of the haptics 30 could be the centre point of the shape of the haptics (in the optical plane of the IOL 21 or optical lens 25). This centre point of the shape of the haptics could be obtained for example by computing the centre of gravity of the shape of the haptics (i.e. weighing each point of the shape equally). The geometrical axis of the haptics 30 or of the IOL 21 is the straight line through the geometrical centre point perpendicular to the optical plane. The haptics 30 are configured to position the IOL 21 in the eye E such that the geometrical centre point is arranged on the centre point C of the limbus L. The haptics 30 are preferably configured to auto-position the IOL 21 in the eye E such that the geometrical centre point of the haptics 30 are centered on the centre point C of the limbus L. Due to the pre-defined orientation of the IOL 21 in the eye E, the directions of the eye E like horizontal direction, nasal direction, temporal direction, vertical direction, upper direction, lower direction will subsequently be used also for the IOL 21 in the sense that the respective direction of the eye applies to the direction of the IOL 21, when implanted in the eye E in the pre-defined orientation. Thus, the nasal direction of the IOL 21 refers to the direction of the IOL 21 which would point in the nasal direction when the IOL 21 is implanted in the eye E of the patient in the pre-defined orientation. Thus, the upper direction of the IOL 21 refers to the direction of the IOL 21 which would point in the upper direction when the IOL 21 is implanted in the eye E of the patient in the pre-defined orientation.

The IOL 21 is configured to be positioned in a pre-defined orientation in the eye E. The pre-defined orientation of the IOL 21 is one rotation state of the IOL 21 around the geometrical axis. In one embodiment, the IOL 21 can only positioned in one orientation by the design of the haptics 30, for example for mutli-point fixation haptics, Iris-Claw haptics, scleral-fixated haptics. In another embodiment, the haptics 30 can allow two or more potentially possible orientations of the IOL 21 in combination of a marking on the IOL 21 which allows the surgeon to position the IOL 21 in the pre-defined orientation in the eye E. Such two or more orientation haptics are for example C-loop haptics, modified C-loop haptics, plate haptics, open-loop haptics. Also other types of haptics 30 might be used.

According to the invention, the optical lens 25 is arranged such with respect to the haptics 30 that the optical centre point of the optical lens 25 is offset horizontally by an horizontal offset and vertically by a vertical offset with respect to the geometrical centre point of the haptics 30 (when the IOL 21 is placed in the eye E in the pre-defined orientation). Preferably, the horizontal offset is preferably nasally, i.e. the optical centre is closer to the nose than the geometrical centre. The horizontal or nasal offset is preferably larger than 0,1 millimetre (mm), preferably than 0,15 mm, preferably than 0,20 mm. The horizontal or nasal offset is preferably smaller than 0,50 millimetre (mm), preferably than 0,40 mm, preferably than 0,35 mm, preferably than 0.30 mm. Preferably, the vertical offset is preferably upwards, i.e. the optical centre is closer to the eyebrows than the geometrical centre. The vertical or upper offset.is preferably larger than 0,05 millimetre (mm), preferably than 0,0,07 mm, preferably than 0,09 mm, preferably than 0,10 mm, preferably than 0,11 mm. The vertical or upper offset is preferably smaller than 0,30 millimetre (mm), preferably than 0,25 mm, preferably than 0,20 mm, preferably than 0,17 mm, preferably than 0,15 mm. Preferably, the horizontal offset is nasally and the vertical offset is upwards, wherein the absolute value of the vertical offset is smaller than the absolute value of the horizontal offset. The nasal and upper offset of the optical centre with respect to the geometrical centre corresponds to the statistcal position of the optical centre and thus would fit in a non-customized IOL more often the optical centre of the eye E of patients than the centred optical centre. However, it has been found out that even for patients with different offsets, this nasal and upper offset brings the benefit of positioniong the IOL closer to the nose and eyebrows which for all patients reduce the probability of the appearance of edge lights on the IOL. It is assumed that the shadow created by the eyebrows and the nose and the close positioning of the IOL to this shadow lead to this beneficial effect independent of the true optical centre of the eye E/pupil P.

The haptics 30 and the optical lens 25 can be integrally formed, e.g. by molding or could also be manufactured separately and then assembled together.

In one embodiment, the IOL according to the invention is preferably a non-customized IOL.

In another embodiment, the IOL is a customizable IOL blank with a pre-defined vertical and horizontal offset of the optical centre of the optical lens 25 with respect to the geometrical centre of the haptics 30 (as described above). The optical lens 25 can be further customized to the measurements of the patients. The optical lens 25 of the blank has however already a certain pre-shape of the lens with an optical centre, e.g. a spheric or aspheric shape of the lens according to a certain power. Therefore, the optical lens 25 can also be called an optical lens blank. The pre-shape of the lens could be further machined, e.g. by mechanical tools, by optical tools (e.g. by laser tools), to obtain the customization of the optical lens 25, e.g. a specific toric lens shape. However, while the optical lens 25 is further customizable, the haptics 30 are well defined and the horizontal and vertical offset is not customizable.

The invention can further refer to a mold for manufacturing an IOL 21 as described above.

Fig. 7 shows a pair of lOLs according to the invention. The pair of IOLs comprise a left IOL 21L and a right IOL 21R. Both IOLs 21L and 21R correspond to an IOL as described above. Preferably, the left IOL21L and the right IOL 21R are different to each other, in particular, the left IOL 21L cannot be rotated by 180° to obtain the right IOL 21R. Preferably, the left IOL 21L has a nasal and upper offset of the optical centre of the optical lens 25 of the left IOL 21L with respect to the geometrical centre of the haptics 30 of the left IOL 21L, and, the right IOL 21R has a nasal and upper offset of the optical centre of the optical lens 25 of the right IOL 21R with respect to the geometrical centre of the haptics 30 of the right IOL 21R. In one embodiment, (the absolute value of) the horizontal offset or nasal offset of the left IOL 21L is the same as (the absolute value of) the horizontal offset or nasal offset of the right IOL 21R. In one embodiment, (the absolute value of) the horizontal offset or nasal offset of the left IOL 21L is different from (the absolute value of) the horizontal offset or nasal offset of the right IOL 21R. In one embodiment, (the absolute value of) the horizontal offset or nasal offset of the left IOL 21L is smaller than (the absolute value of) the horizontal offset or nasal offset of the right IOL 21R. The differences could correspond to statistical differences in the offset of the optical centre C1/C2 for the right and left eye. In one embodiment, (the absolute value of) the vertical offset or upper offset of the left IOL 21L is the same as (the absolute value of) the vertical offset or upper offset of the right IOL 21R. In one embodiment, (the absolute value of) the vertical offset or upper offset of the left IOL 21L is different from (the absolute value of) the vertical offset or upper offset of the right IOL 21R. In one embodiment, (the absolute value of) the vertical offset or upper offset of the left IOL 21L is larger than (the absolute value of) the vertical offset or upper offset of the right IOL 21R. The differences could correspond to statistical differences in the offset of the optical centre C1 /C2 for the right and left eye.

The pair of lOLs 21L, 21R are preferably non-costomized IOLs. The pair of lOLs 21L, 21R could also be a pair of customizable IOL blanks as described above.

Multiple equal/identical pairs of IOLs 21L, 21R could be combined to sets of two, three or more pairs. Such a set of equal/identical pairs with N pairs; N being a natural number larger than 1, would have N left IOLs 21L and N right IOLs 21R, wherein all N right IOLs 21R are equal to each other and wherein all N left lOLs 21L are equal to each other (while the right IOLs 21R and the left lOLs 21L are different from each other).

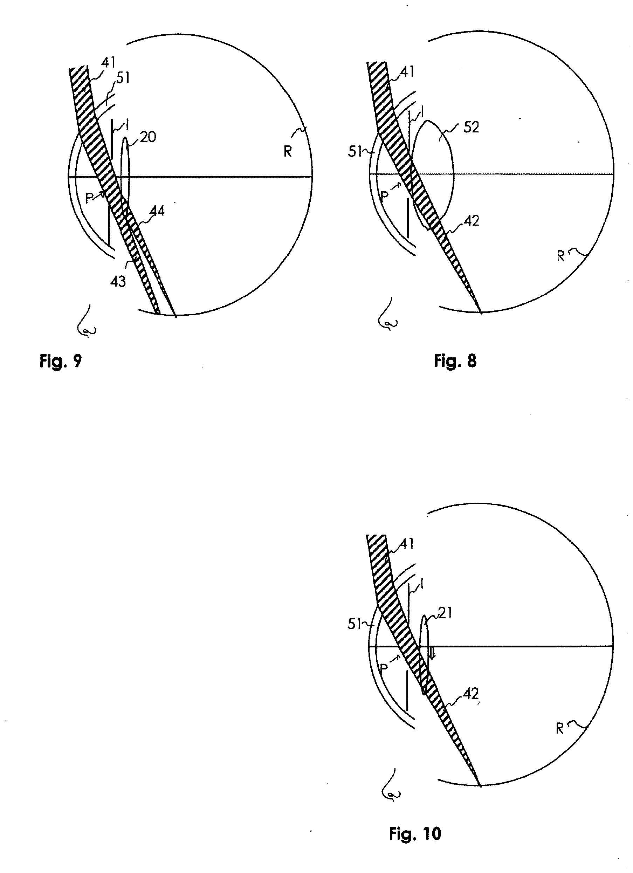

In order to show the beneficial effect of the IOL with a standard nasal and/or upwards offset for dysphotopsia, this offset placement in Fig. 10 is compared with an eye with a natural lens 52 in Fig. 8 and an eye with an implanted IOL 20 from the state of the art.

In Fig. 8, oblique light rays 41 from a temporal side of the face are focused by the cornea 51 through the pupil P on the lens 52 which focuses this oblique temporal light rays 41 through the natural lens 52 focused on the retina R of the eye E.

In Fig. 9, the natural lens 52 has been replaced by a surgery with an IOL 20 according to the state of the art whose optical centre point is aligned with the geometrical centre point of the IOL 20. Thus, the optical centre point of the optical lens 25 of the IOL 20 is aliged with the centre point of the limbus L or the iris I. We assume in Fig. 9 that the pupil centre point C is aligned with the limbus centre point and thus with the optical centre point of the IOL 20. Any IOL 20 is much smaller than the natural lens 52. Therefore, the same temporal oblique light ray 41 which was still focused by the natural lens 52 in Fig. 8 hits now on the nasally arranged edge of the IOL 20. Thus, a first portion of the light ray 41 is now focused by the peripheral part of the IOL 20 leading to a first light ray 44 focused by the IOL 20 and a second light ray 43 not passing through the IOL 20. This leads thus to errors in the image on the retina R. This effect would be even worse, when the pupil centre P of the eye would be nasally offset as the second light ray 43 would be even larger. This effect is even worse for phakic lOLs as they would be positioned even more to the interior of the eye.

Fig. 10 shows now the same eye with an IOL 21 according to the invention where the optical centre point of the optical lens 25 is offset nasally. Since the optical lens 25 of the IOL 21 is moved nasally, the temporal oblique light rays 41 are not hitting the edge of the IOL 21 any more and again as for the natural lens 52, the light 42 is focused on one single point on the retina R. Thus, the nasal offset helps even for persons without nasal offset of the pupil centre point to see /experience dysphotopsia. One could argue that however now nasally oblique light rays are more likely to generate dysphotopsia on the nasal side of the vision as the optical lens 25 of the IOL 21 is moved nasally and thus, there is a smaller portion of the optical lens 25 of the IOL 21 on the temporal side of the pupil P. However, the (absolute value of the) maximum nasal oblique light ray angles are much larger than the (absolute value of the) maximum temporal oblique light ray angles, because the nose blocks the light rays of the larger oblique light ray angles. Oblique light ray angles are here defined from the optical axis of the eye E so that the larger the (absolute value of the) light ray angle gets, the more oblique is the light ray coming on the eye E. Therefore, the nasal offset of the optical lens 25 with respect to the pupil centre point C in eyes which do not show such an nasal offset of the pupil centre point C surprisingly has a beneficial effect for the dysphotopsia.

Thus, the nasal offset of the optical centre point of the optical lens 25 with respect to the geometrical centre point of the haptics 30 has surprisingly a beneficial effect to avoid dysphotopsia for all patients, the ones which actually show a nasal offset of the pupil centre and also for the ones which do not show such a nasal offset of their pupil centre. Therefore, the standard nasal offset of the optical lens 25 with respect to the geometrical centre point of the haptics 30 for all patients surprisingly avoids dysphotopsia even though the optical centre point of the optical lens 25 of the IOL 21 is not necessarily aligned with the centre point C1m C2 of the pupil P1, P2.

The same argumentation can be made for the offset of the optical lens 25 of the IOL 21 upwards, as this moves the IOL 21 closer to the eyebrow so that dysphotopsia generated from oblique light rays from the lower field of vision can be effectively avoided, while the problem of oblique light rays from the upper field of vision is less problematic due to the shadow generated from the eyebrows. Therefore, either the nasal offse or the upwards offset alone would also reduce the problem of dysphotopsia, but obviously the combination of both offsets reduces the problem even more. It should be understood that the present invention is not limited to the described embodiments and that variations can be applied without going outside of the scope of the claims.

## Claims

1. Non-customized intraocular lens comprising haptics configured to position the intraocular lens in a pre-defined orientation in the eye, wherein the haptics define a geometrical centre point of the intraocular lens, wherein the intraocular lens comprises further an optical lens with an optical centre point through which the optical axis of the optical lens extends, wherein the optical lens is arranged such with respect to the haptics that the optical centre point is offset horizontally and vertically with respect to the geometrical centre point.

2. Non-customized intraocular lens according to claim 1, wherein the optical centre point is offset nasaly with respect to the geometrical centre point.

3. Non-customized intraocular lens according to claim 2, wherein the optical centre point is offset nasaly with respect to the geometrical centre point by an nasal offset larger than 0,1mm and/or smaller than 0,35mm.

4. Non-customized intraocular lens according to one of claims 1 to 3, wherein the optical centre point is upwards with respect to the geometrical centre point.

5. Non-customized intraocular lens according to claim 4, wherein the optical centre point is offset upwards with respect to the geometrical centre point by a vertical offset larger than 0,05mm and/or smaller than 0,25mm.

6. Non-customized intraocular lens according to one of claims 1 to 5, wherein the optical centre point is offset nasaly and upwards with respect to the geometrical centre point, wherein the nasal offset is larger than the upwards offset.

7. Customizable intraocular lens blank comprising haptics configured to position the intraocular lens in a pre-defined orientation in the eye, wherein the haptics define a geometrical centre point of the intracolar lens, wherein the intraocular lens comprises further an optical lens blank with an optical centre point through which the optical axis of the optical lens blank extends, wherein the optical lens blank is configured to be customizable to a patient wherein the optical lens blank is arranged such with respect to the haptics that the optical centre point is offset horizontally and vertically with respect to the geometrical centre point.

8. Customized intraocular lens comprising haptics configured to position the intraocular lens in a pre-defined orientation in the eye, wherein the haptics define a geometrical centre point of the intracolar lens, wherein the intraocular lens comprises further an optical lens with an optical centre point through which the optical axis of the optical lens extends, wherein the optical lens is customized to a patient, wherein the optical lens is arranged such with respect to the haptics that the optical centre point is offset horizontally and vertically with respect to the geometrical centre point, wherein the offset arrangement of the optical centre point of the optical lens with respect to the geometrical centre of the haptics is non-costumized.

9. Pair of intraocular lenses comprising a left intraocular lens and a right intraocular lens,
wherein the left intraocular lens comprises left haptics configured to position the left intraocular lens in a defined orientation in a left eye of a patient, wherein the left haptics define a left geometrical centre point of the left intracolar lens, wherein the left intraocular lens comprises further a left optical lens with a left optical centre point through which the optical axis of the left optical lens extends, wherein the left optical lens is arranged such with respect to the left haptics that the left optical centre point is offset horizontally and vertically with respect to the left geometrical centre point,
wherein the right intraocular lens comprises right haptics configured to position the right intraocular lens in a defined orientation in a right eye of the patient, wherein the right haptics define a right geometrical centre point of the right intracolar lens, wherein the right intraocular lens comprises further a right optical lens with a right optical centre point through which the optical axis of the right optical lens extends, wherein the right optical lens is arranged such with respect to the right haptics that the right optical centre point is offset horizontally and vertically with respect to the right geometrical centre point,
wherein the right intraocular lens and the left intraocular lens are intraocular lenses with a non-customized offset, preferably non-customized intraocular lenses or customizable intraocular lens blanks or customized intraocular lenses with non-customized offset.

10. Pair according to claim 9, wherein the left intraocular lens is different from the right intraocular lens such that the right intralcular lens cannot be obtained by a rotation by 180° of the left intraocular lens around the left geometrical centre point.

11. Pair according to claim 9 or 10, wherein the left optical centre point is offset nasaly and upwards with respect to the left geometrical centre point, wherein the right optical centre point is offset nasaly and upwards with respect to the right geometrical centre point.

12. Pair according to one of claims 9 to 11, wherein the nasal offset of the left optical centre point is different from the nasal offset of the right optical centre point and/or the vertical offset of the left optical centre point is different from the vertical offset of the rigt optical centre point.

13. Pair according to claim 12. wherein the nasal offset of the left optical centre point is smaller than the nasal offset of the right optical centre point and/or the vertical offset of the left optical centre point is larger than the vertical offset of the rigt optical centre point.

14. Multiple pairs comprising two, three or more pairs according to one of claims 10 to 13, wherein the two, three or more pairs are identical pairs.

15. Method for selecting an intraocular lens according to one of claims 1 to 8 for a patient or for selecting a pair of intraocular lenses according to one of claims 8 to 13 for a patient, wherein the intraocular lens or the pair is selected independent from the pupil centre of the patient.
